# EUROPEAN PATENT APPLICATION

(11) **EP 4 471 148 A1**
(43) Date of publication of application: **04.12.2024**
(21) Application number: 22923633.6
(22) Date of filing: 28.12.2022
(51) Int. Cl.: C12N 15/64, C12N 1/21, C12P 13/08, C12R 1/15

(54) **PROMOTER, THREONINE-PRODUCING RECOMBINANT MICROORGANISM AND USE THEREOF**

(30) Priority: 26.01.2022 CN 202210094640
(71) Applicant: Langfang Meihua Biotechnology Development Co., Ltd, Langfang City, Hebei 065001 (CN)
(72) Inventor: KANG, Pei, Langfang City, Hebei 065001 (CN); WANG, Zhiquan, Langfang City, Hebei 065001 (CN); GONG, Weibo, Langfang City, Hebei 065001 (CN); HE, Jun, Langfang City, Hebei 065001 (CN); LI, Yan, Langfang City, Hebei 065001 (CN)
(74) Representative: CH Kilger Anwaltspartnerschaft mbB
(86) International application number: PCT/CN2022/142851
(87) International publication number: WO 2023/142848

(57) **Abstract**

Provided is a promoter, which has a nucleotide sequence shown in SEQ ID NO. 1. The promoter is obtained by means of rationally designing the promoter of a dapA gene, has obviously reduced transcriptional activity and can be used for the weakened expression of microorganism genes. Substituting the original promoters of the dapA gene, a ddh gene, an ilvA gene, a tdcB gene and a gltA gene in corynebacterium glutamicum by using the promoter enables the expression levels of these genes to be significantly reduced, the carbon metabolic in a recombinant microorganism constructed thereby flows more to a threonine anabolism pathway, and the recombinant microorganism has a significantly improved production of threonine in comparison with an original strain and has good growth performance.

## Description

### Technical Field

The present invention relates to the technical field of microbial engineering, specifically to promoters, threonine-producing recombinant microorganisms and their uses.

### Background Art

L-Threonine has a chemical name of β-hydroxy-α-aminobutyric acid, with a molecular formula of C₄H₉NO₃, and a relative molecular mass of 119.12. L-threonine is an essential amino acid. Threonine is mainly used in medicine, chemical reagents, food fortifiers, feed additives, etc.

In Corynebacterium glutamicum, the production of threonine from oxaloacetate requires a five-step catalytic reaction. The catalytic enzymes involved in the five-step reaction are aspartate kinase (encoded by lysC), aspartate semialdehyde dehydrogenase (encoded by asd), homoserine dehydrogenase (encoded by horn), homoserine kinase (encoded by thrB) and threonine synthase (encoded by thrC). Regarding the lysC gene and horn gene, there have been reports of horn genes and lysC genes that are deregulated (Reinscheid D J, Eikmanns B J, Sahm H. Analysis of a Corynebacterium glutamicum horn gene coding for a feedback-resistant homoserine dehydrogenase. [J]. Journal of Bacteriology, 1991, 173(10):3228-3230; Eikmanns B J, Eggeling L, Sahm H. Molecular aspects of lysine, threonine, and isoleucine biosynthesis in Corynebacterium glutamicum.[J]. Antonie Van Leeuwenhoek, 1993, 64(2):145-163.). At present, there are some reports on the construction of engineered bacteria for fermentative production of threonine using Corynebacterium glutamicum, for example: Lothar Eggling et al. increased the threonine production from 49 mM to 67 mM by weakening the coding gene glyA in the threonine utilization pathway and overexpressing the threonine export protein ThrE (Simic P, Willuhn J, Sahm H, et al. Identification of glyA (Encoding Serine Hydroxymethyltransferase) and Its Use Together with the Exporter ThrE To Increase L-Threonine Accumulation by Corynebacterium glutamicum[J]. Applied and Environmental Microbiology, 2002, 68(7):3321-3327.).

Current reports on using Corynebacterium glutamicum to produce threonine mainly focus on the metabolic engineering modification of the threonine synthesis pathway, while there are fewer reports on the optimization of carbon metabolic flow. In addition, when regulating gene expression in Corynebacterium glutamicum, the enhanced expression of genes mainly uses synthetic strong promoters, strong promoters of endogenous genes or the introduction of heterologous strong promoters. However, there are relatively few reports on promoters that weaken gene expression. Weakening the expression of a gene mainly relies on the replacement of start codons and gene knockout technology.

### Summary of the Invention

The objective of the present invention is to provide a mutated promoter of dapA gene. Another objective of the present invention is to provide a recombinant microorganism constructed by using the promoter for gene expression regulation and the use thereof.

Specifically, the present invention provides the following technical solutions:
The present invention provides a promoter having a nucleotide sequence as shown in SEQ ID NO. 1.

The promoter described above is a mutated dapA gene (4-hydroxy-tetrahydropyridinedicarboxylate synthase gene) promoter (mPdapA), which has the activity of initiating gene transcription, but the activity of the promoter compared to the wild type is significantly reduced

Preferably, the nucleotide sequence of the above mutated dapA gene promoter is shown as in SEQ ID NO.1.

The present invention further provides a biological material, which comprises the mutated dapA gene promoter. The biological material is a recombinant DNA, a vector or a host cell.

The recombinant DNA described above may be a recombinant DNA obtained by operably linking a gene of interest downstream of the promoter, or a recombinant DNA obtained by operably linking a gene of interest downstream of the promoter, and operably linking other transcriptional regulatory elements and translation regulatory elements upstream or downstream of the promoter, or a recombinant DNA obtained by operably linking a homologous arm fragment for homologous recombination upstream and/or downstream of the promoter.

The vector described above may be a plasmid vector, a viral vector or a transposon.

The host cell described above is preferably a microbial cell.

The microbial cell is preferably a bacterium of the genus Escherichia or Corynebacterium. Among them, the Escherichia bacterium is preferably Escherichia coli; and the bacteria of the genus Corynebacterium are preferably Corynebacterium glutamicum, Corynebacterium pekinense, Corynebacterium effeciens, Corynebacterium crenatum, Corynebacterium thermoaminogenes or Corynebacterium aminogenes.

The present invention provides the use of the mutated promoter of dapA gene described above in expressing of a gene of interest.

The above use is specifically: the gene of interest is operably linked to the mutant promoter of dapA gene to obtain a recombinant DNA, which is then introduced into a host cell to express the gene of interest.

The mutated dapA promoter described above can be used to drive the expression of metabolism-related genes of the metabolite of interest, and can be particularly used to reduce the expression of competing pathway-related genes of the metabolite of interest to increase the accumulation of the metabolite of interest.

The present invention further provides the use of the mutated dapA gene promoter described above in improving the production or yield of a microbial metabolite, or in constructing a production strain of a microbial metabolite.

Preferably, the metabolite is threonine or a derivative thereof.

In the threonine fermentation, lysine and isoleucine are the main by-products, and the lysine and isoleucine synthesis pathways are the main competing pathways for the threonine synthesis pathway. Therefore, it is necessary to block or weaken by-product pathways such as lysine synthesis pathway, so that more carbon metabolic flow will go to threonine synthesis pathway. However, direct knockout of lysine synthesis gene dapA and other genes will lead to a sharp decline in the growth rate of strains or cause nutrient defects in cells, which is not good to the fermentative production of threonine. According to the present invention, the mutated dapA gene promoter described above is used to weaken the lysine and isoleucine synthesis genes, which can avoid the generation of nutrient defects, significantly improve the allocation of carbon metabolic flow towards the threonine synthesis pathway and ensure the normal growth of the strain at the same time, enhance the threonine synthesis ability of the strain, and reduce the accumulation of lysine and isoleucine.

Based on the above findings, the present invention provides a recombinant microorganism in which a gene encoding an enzyme selected from at least one of the following (1) to (4) is driven by the mutated dapA gene promoter described above for transcription:
(1) 4-hydroxy-tetrahydrodipicolinate synthase;
(2) diaminopimelate dehydrogenase;
(3) threonine dehydratase;
and (4) citrate synthase.

As an embodiment of the present invention, the genes encoding 4-hydroxy-tetrahydrodipicolinate synthase and diaminopimelate dehydrogenase are driven by the mutated dapA gene promoter described above for transcription in the recombinant microorganism.

As another embodiment of the present invention, the genes encoding 4-hydroxy-tetrahydrodipicolinate synthase, diaminopimelate dehydrogenase, and threonine dehydratase are driven by the mutated dapA gene promoter described above for transcription in the recombinant microorganism.

As another embodiment of the present invention, the genes encoding 4-hydroxy-tetrahydrodipicolinate synthase, diaminopimelate dehydrogenase, threonine dehydratase, and citrate synthase are driven by the mutated dapA gene promoter described above for transcription in the recombinant microorganism.

The 4-hydroxy-tetrahydrodipicolinate synthase, diaminopimelate dehydrogenase, threonine dehydratase and citrate synthase described above have reference sequence numbers of WP_011014792.1, WP_011015254.1, WP_011014022.1, WP_003862033.1 on NCBI, respectively, or amino acid sequences with 90% similarity to and equivalent function as the above reference sequences.

The above-mentioned "driven by the mutated dapA gene promoter described above for transcription" is specifically to replace the original promoter of the gene with the mutated dapA gene promoter.

The expression of 4-hydroxy-tetrahydrodipicolinate synthase, diaminopimelate dehydrogenase, threonine dehydratase and citrate synthase in the recombinant microorganism is significantly reduced, the synthesis ability of threonine, the production and yield of threonine are significantly higher than that of the original strain, and the accumulation of by-products is significantly reduced.

Due to the rigorous metabolic regulation of microorganisms, aspartate kinase and homoserine dehydrogenase in the threonine synthesis pathway are strictly regulated by intracellular threonine concentration. Therefore, in the original strain that can accumulate threonine, the threonine synthesis pathway is firstly unlocked, mainly including the deregulation or the enhancement of enzyme activity of aspartate kinase and homoserine dehydrogenase.

Specifically, in the recombinant microorganism, the enzyme activity of aspartate kinase and/or homoserine dehydrogenase is enhanced and/or deregulated thereof.

The aspartate kinase and homoserine dehydrogenase have reference sequence numbers of WP_003855724.1 and WP_003854900.1 on NCBI, respectively, or amino acid sequences with 90% similarity to and equivalent function as the above reference sequences.

Preferably, the microorganism is any of the following (1) to (7):
(1) microorganisms in which the gene encoding 4-hydroxy-tetrahydrodipicolinate synthase is driven by the mutated dapA gene promoter described above, and the enzyme activity of aspartate kinase and/or homoserine dehydrogenase is enhanced and/or deregulated;
(2) microorganisms in which the gene encoding diaminopimelate dehydrogenase is driven by the mutated dapA gene promoter described above, and the enzyme activity of aspartate kinase and/or homoserine dehydrogenase is enhanced and/or deregulated;
(3) microorganisms in which the gene coding threonine dehydratase is driven by the mutated dapA gene promoter described above, and the enzyme activity of aspartate kinase and/or homoserine dehydrogenase is enhanced and/or deregulated;
(4) microorganisms in which the gene encoding citrate synthase is driven by the mutated dapA gene promoter described above, and the enzyme activity of aspartate kinase and/or homoserine dehydrogenase is enhanced and/or deregulated;
(5) microorganisms in which the genes encoding 4-hydroxy-tetrahydrodipicolinate synthase and diaminopimelate dehydrogenase are driven by the mutated dapA gene promoter described above, and the enzyme activity of aspartate kinase and/or homoserine dehydrogenase is enhanced and/or deregulated;
(6) microorganisms in which the genes encoding 4-hydroxy-tetrahydrodipicolinate synthase, diaminopimelate dehydrogenase and threonine dehydratase are driven by the mutated dapA gene promoter described above, and the enzyme activity of aspartate kinase and/or homoserine dehydrogenase is enhanced and/or deregulated;
(7) microorganisms in which the genes encoding 4-hydroxy-tetrahydrodipicolinate synthase, diaminopimelate dehydrogenase, threonine dehydratase, and citrate synthase are driven by the mutated dapA gene promoter described above, and the enzyme activity of aspartate kinase and/or homoserine dehydrogenase is enhanced and/or deregulated.

Preferably, the enhancement of the enzyme activity is achieved by any one selected from the following 1) to 6), or an optional combination thereof:
1) enhancement by introduction of a plasmid having the gene encoding the enzyme;
2) enhancement by increasing the copy number of the gene encoding the enzyme on the chromosome;
3) enhancement by altering the promoter sequence of the gene encoding the enzyme on the chromosome;
4) enhancement by operably linking a strong promoter to the gene encoding the enzyme;
5) enhancement by altering the amino acid sequence of the enzyme; and
6) enhancement by altering the nucleotide sequence encoding the enzyme.

For Corynebacterium glutamatum, the threonine dehydratase is encoded by the ilvA and tdcB genes.

Further preferably, the enhancement of the enzyme activity is achieved by replacing the original promoter of the gene encoding the enzyme with a strong promoter.

The strong promoter is preferably Psod or PcspB.

Among them, the enhancement of the enzyme activity of aspartate kinase is preferably achieved by replacing the original promoter of the gene encoding aspartate kinase with a Psod strong promoter and mutating the start codon of the gene to an ATG.

The enhancement of the enzyme activity of homoserine dehydrogenase is preferably by replacing the original promoter the gene encoding homoserine dehydrogenase with a PcspB strong promoter.

Feedback-resistant aspartate kinase is preferably achieved by engineering aspartate kinase to undergo a T311I mutation.

Feedback-resistant homoserine dehydrogenase is preferably achieved by engineering homoserine dehydrogenase to undergo a G378E mutation.

The recombinant microorganism of the present invention is preferably Corynebacterium bacteria; more preferably Corynebancterium glutamicum. Corynebacterium glutamatum includes ATCC13032, ATCC13870, ATCC13869, ATCC21799, ATCC21831, ATCC14067, ATCC13287, etc. (see NCBI Corunebacterium glutamicum evolutionary tree https://www.ncbi.nlm.nih.gov/genome/469), more preferably Corynebacterium glutamatum ATCC 13032.

The present invention further provides a method for constructing the recombinant microorganism described above, the method comprises replacing the promoter of the gene encoding at least one of 4-hydroxy-tetrahydrodipicolinate synthase, diaminopimelate dehydrogenase, threonine dehydratase and citrate synthase with the mutated dapA gene promoter described above.

Preferably, the method further comprises: enhancing the enzyme activity of aspartate kinase and/or homoserine dehydrogenase and/or obtaining feedback-resistant aspartate kinase and/or homoserine dehydrogenase;
wherein, the enhancement of the enzyme activity is achieved by any one selected from the following 1) to 6), or an optional combination thereof:
1) enhancement by introduction of the plasmid having a gene encoding the enzyme;
2) enhancement by increasing the copy number of the gene encoding the enzyme on the chromosome;
3) enhancement by altering the promoter sequence of the gene encoding the enzyme on the chromosome;
4) enhancement by operably linking a strong promoter to the gene encoding the enzyme;
5) enhancement by altering the amino acid sequence of the enzyme; and
6) enhancement by altering the nucleotide sequence encoding the enzyme.

The above-mentioned modification methods of related strains, including gene enhancement, are all modification methods known to those skilled in the art, see Man Zaiwei. Systemic pathway engineering modification of Corynebacterium crenatum to produce L-arginine with high yield [D]. Jiangnan University, 2016; Cui Yi. Metabolic engineering modification of Corynebacterium glutamicum to produce L-leucine [D]. Tianjin University of Science and Technology.; Xu Guodong. Construction of L-isoleucine producing strain and optimization of fermentation conditions. Tianjin University of Science and Technology, 2015.

The present invention further provides any of the following uses of the recombinant microorganism:
(1) use in fermentative production of a microbial metabolite or a derivative thereof;
(2) use in breeding a production strain of a microbial metabolite or a derivative thereof; and
(3) use in increasing the production and/or yield of a microbial metabolite.

Preferably, the microbial metabolite is threonine.

The present invention further provides a method for fermentative production of threonine or a derivative thereof, wherein the method comprises the steps of cultivating the recombinant microorganism and obtaining threonine or a derivative thereof by isolating from the culture.

Specifically, the above method comprises that: the recombinant microorganism is inoculated in a seed medium to obtain a seed liquid, the seed liquid is inoculated in a fermentation medium for culture to obtain a fermentation liquid, and the fermentation liquid is separated and extracted to obtain threonine or a derivative thereof.

Preferably, the fermentation medium comprises the following components: corn steep liquor 45-55 mL/L, glucose 25-35 g/L, ammonium sulfate 3-5 g/L, MOPS 25-35 g/L, potassium dihydrogen phosphate 8-12 g/L, urea 15-25 g/L, biotin 8-12 mg/L, magnesium sulfate 5-7 g/L, ferrous sulfate 0.5-1.5 g/L, V_{B1}•HCl 35-45 mg/L, calcium pantothenate 45-55 mg/L, nicotinamide 35-45 mg/L, manganese sulfate 0.5-1.5 g/L, zinc sulfate 15-25 mg/L, and copper sulfate 15-25 mg/L, pH 7.0-7.2.

The beneficial effects of the present invention are as follows: The present invention obtains a mutated dapA gene promoter with significantly reduced transcription activity by rationally designing the promoter of the dapA gene, and the promoter can be used for weakened expression of genes.

The promoter is used to replace the original promoter of the gene encoding 4-hydroxy-tetrahydrodipicolinate synthase, diaminopimelate dehydrogenase, threonine dehydratase and citrate synthase in Corynebacterium glutamicum, so that the expression levels of these genes are significantly reduced. The carbon metabolic in the recombinant microorganism thereby flows more towards the threonine anabolic pathway, and the ability of the strain to produce threonine is significantly improved. The threonine production is increased by 279% compared to the original strain, and at the same time, the strain has good growth performance.

### Detailed Description of Embodiments

The following examples are intended to illustrate the present invention but are not intended to limit the scope of the present invention.

Details of the genes and enzymes involved in the following examples are as follows:
aspartate kinase, coding gene lysC, NCBI number: cg0306, Cgl0251, NCgl0247.
homoserine dehydrogenase, coding gene horn, NCBI number: cg1337, Cgl1183, NCgl1136.
4-hydroxy-tetrahydrodipicolinate synthase, coding gene dapA, NCBI number: cg2161, Cgl1971, NCgl 1896.
diaminopimelate dehydrogenase, coding gene ddh, NCBI number: cg2900, Cgl2617, NCgl2528.
threonine dehydratase, coding gene ilvA, NCBI number: cg2334, Cgl2127, NCgl2046.
threonine dehydratase, coding gene tdcB, NCBI number: cg1116, Cgl0978, NCgl0939.
citrate synthase, coding gene gltA, NCBI number: cg0949, Cgl0829, NCgl0795.

The present invention constructed a threonine producing strain through metabolic engineering modification on the basis of a wild type strain of Corynebacterium glutamicum ATCC13032. First, the synthesis pathway of threonine was unlocked, mainly including the feedback-resistant or the enhancement of expression of aspartate kinase (lysC) and homoserine dehydrogenase (horn). Specifically, the expression of aspartate kinase was enhanced in Corynebacterium glutamatum ATCC13032 and the regulation of theenzyme was relieved by mutation to obtain a engineered strain SMCT136. On this basis, the expression of homoserine dehydrogenase was enhanced and deregulated by mutation to obtain an modified strain SMCT137 which has preliminary threonine synthesis ability with the production of 2.4 g/L.

For the engineered SMCT137, lysine and isoleucine are the main by-products during the fermentation of threonine, and the lysine and isoleucine synthesis pathways are the main competing pathways for the threonine synthesis pathway. Therefore, it is necessary to block or weaken by-product pathways such as lysine synthesis pathway, so that more carbon metabolic will flow to threonine synthesis pathway. However, direct knockout of lysine synthesis genes such as dapA will lead to a sharp decline in the growth rate of strains or cause nutrient defects in cells, which is not conducive to the fermentation of threonine. Therefore, the present invention adopts the method of rationally modifying the promoter of dapA gene and obtain a mutated dapA gene promoter (mPdapA) by rationally designs and screens, in order to weaken the expression level of 4-hydroxy-tetrahydrodipicolinate synthase, so as to achieve the purpose of giving consideration to cell growth and carbon flow optimization. On the basis of SMCT137, a site-directed mutation was performed on the promoter of the gene dapA encoding 4-hydroxy-tetrahydrodipicolinate synthase of the lysine synthesis pathway (replacing the original dapA gene promoter with the mutated dapA promoter) to obtain strain SMCT138. The strain had a reduced expression level of 4-hydroxy-tetrahydrodipicolinate synthase and an increased ability to produce threonine from 2.4 g/L to 3.0 g/L, and the by-product lysine was reduced from 1.1 g/L to 0.8 g/L.

In addition, the present invention also uses SMCT137 as the original strain, and uses the mutated dapA promoter to replace the promoters of ddh, ilvA, tdcB, and gltA, respectively, to obtain engineered strain SMCT139, SMCT140, SMCT141, and SMCT142. It was verified that the expression levels of the corresponding proteins diaminopimelate dehydrogenase, threonine dehydratase (ilvA), threonine dehydratase (tdcB), and citrate synthase were reduced, and the replacement of the promoter of ddh reduced the lysine content from 1.1 g/L to 0.9 g/L, and increased the threonine production by 0.4 g/L; The replacement of the promoters of ilvA and tdcB reduced the isoleucine content by 0.6 g/L and 0.2 g/L, respectively, and increased the threonine production by 0.8 g/L and 0.6 g/L, respectively; the replacement of the promoter of gltA increased the threonine production by 0.8 g/L.

Further, On the basis of the above-mentioned individual weakening, the modification of the above-mentioned sites was integrated into SMCT138 step by step to obtain the modified strain SMCT143, SMCT144, SMCT145, and SMCT146. The threonine production of the engineered strain SMCT143 increased by 20%, and the byproduct lysine decreased from 0.8 g/L to 0.4 g/L. The threonine production of the engineered strain SMCT144 increased by 67%, and the byproduct isoleucine decreased from 1.3 g/L to 0.6 g/L. The threonine production of the engineered strain SMCT145 increased by 113%, and the byproduct isoleucine decreased from 0.6 g/L to 0.3 g/L; the threonine production of the engineered strain SMCT146 increased by 203%.

The construction process of the modified strain and the performance of the strain in the fermentative production of threonine are described in detail below by means of examples.

### Example 1 Construction of plasmids for genome modification of strains

### 1. Construction of the plasmid pK18mobsacB-Psod-lysC^{gla-T3llI} for enhancing expression of aspartate kinase

The upstream homologous arm up was obtained by PCR amplification with P21/P22 primer pair using ATCC13032 genome as template, the promoter fragment Psod was obtained by PCR amplification with P23/P24 primer pair, lysC^{gla-T3llI} was obtained by PCR amplification with P25/P26 primer pair, and the downstream homologous arm dn was obtained by PCR amplification with P27/P28 primer pair. The up-Psod fragment was obtained by fusion PCR with P21/P24 primer pair using up and Psod as templates. The full-length fragment up-Psod-lysC^{gla-T3llI}-dn was obtained by fusion PCR with P21/P28 primer pair using up-Psod, lysC^{glaT3llI} and dn as templates. pK18mobsacB was digested with BamHI/HindIII. The two were assembled using a seamless cloning kit and transformed into Trans1 T1 competent cells to obtain the recombinant plasmid pK18mobsacB-Psod-lysC^{gla-T3llI}.

### 2. Construction of plasmid pK18mobsacB-PcspB-hom^{GapG378E} for enhanced expression of homoserine dehydrogenase

The plasmid construction method was referred to 1, and the primers used were P29, P30, P31, P32, P33, P34, P35, and P36.

### 3. Construction of plasmid pK18mobsacB-ΔPdapA:: mPdapA for 4-hydroxy-tetrahydrodipicolinate synthase promoter replacement

The mutated dapA promoter mPdapA (with a sequence as shown in SEQ ID NO.1) was synthesized artificially. The mPdapA promoter fragment was obtained by PCR amplification with PW21/22 primer pair using the synthetic mPdapA sequence as template. The upstream homologous arm up was obtained by PCR amplification with PW1/2 primer pair using ATCC13032 genome as template; the upstream homologous arm dn was obtained by PCR amplification with PW3/4 primer pair; and the fragment up-mPdapA-dn was obtained by fusion PCR with PW1/4 primer pair using up, mPdapA, and dn as templates. pK18mobsacB was digested with BamHI/HindIII. The two were assembled using a seamless cloning kit and transformed into Trans1 T1 competent cells to obtain the recombinant plasmid pK18mobsacB-ΔPdapA::mPdapA.

### 4. Construction of plasmid pK18mobsacB-ΔPddh::mPdapA for diaminopimelate dehydrogenase promoter replacement

The plasmid construction method was referred to 3, and the primers used were PW5, PW6, PW7, PW8, PW21, and PW22.

### 5. Construction of plasmidpK18mobsacB-AilIvA::mPdapA for threonine dehydratase (ilvA) promoter replacement

The plasmid construction method referred to 3, and the primers used were PW9, PW10, PW11, PW12, PW21, and PW22.

### 6. Construction of plasmid pK18mobsacB-ΔPtdcB::mPdapA for threonine dehydratase (tdcB) promoter replacement

The plasmid construction method was referred to 3, and the primers used were PW13, PW14, PW15, PW16, PW21, and PW22.

### 7. Construction of plasmid pK18mobsacB-ΔPgltA::mPdapA for citrate synthase promoter replacement

The plasmid construction method referred to 3, and the primers used were PW17, PW18, PW19, PW20, PW21, and PW22.

The primers used in the above plasmid construction process are shown in Table 1.

**Table 1 Primer sequences**

| Name | Sequence |
|---|---|
| P21 | AATTCGAGCTCGGTACCCGGGGATCCAGCGACAGGACAAGCACTGG |
| P22 | CCCGGAATAATTGGCAGCTATGTGCACCTTTCGATCTACG |
| P23 | CGTAGATCGAAAGGTGCACATAGCTGCCAATTATTCCGGG |
| P24 | TTTCTGTACGACCAGGGCCATGGGTAAAAAATCCTTTCGTA |
| P25 | TACGAAAGGATTTTTTACCCATGGCCCTGGTCGTACAGAAA |
| P26 | TCGGAACGAGGGCAGGTGAAGGTGATGTCGGTGGTGCCGTCT |
| P27 | AGACGGCACCACCGACATCACCTTCACCTGCCCTCGTTCCGA |
| P28 | GTAAAACGACGGCCAGTGCCAAGCTTAGCCTGGTAAGAGGAAACGT |
| P29 | AATTCGAGCTCGGTACCCGGGGATCCCTGCGGGCAGATCCTTTTGA |
| P30 | ATTTCTTTATAAACGCAGGTCATATCTACCAAAACTACGC |
| P31 | GCGTAGTTTTGGTAGATATGACCTGCGTTTATAAAGAAAT |
| P32 | GTATATCTCCTTCTGCAGGAATAGGTATCGAAAGACGAAA |
| P33 | TTTCGTCTTTCGATACCTATTCCTGCAGAAGGAGATATAC |
| P34 | TAGCCAATTCAGCCAAAACCCCCACGCGATCTTCCACATCC |
| P35 | GGATGTGGAAGATCGCGTGGGGGTTTTGGCTGAATTGGCTA |
| P36 | GTAAAACGACGGCCAGTGCCAAGCTTGCTGGCTCTTGCCGTCGATA |
| PW1 | CATGATTACGAATTCGAGCTCGGTACCCGGGGATCCTCCGCGACCCATGAATT GGT |
| PW2 | ACTATATGAATTTTTAGCTCTTGGGTGTGAGCTTTGCGTTAAAAGTCCAT |
| PW3 | TGAGCACAGGTTTAACAGCTAAGACCGGAGTAGAGCACTTCGGCACCGTTG |
| PW4 | TCACGACGTTGTAAAACGACGGCCAGTGCCAAGCTTACCCTTGGCGTCCTTGA CCG |
| PW5 | CATGATTACGAATTCGAGCTCGGTACCCGGGGATCCGTATCGGCAGTTCCAGC CAT |
| PW6 | ACTATATGAATTTTTAGCTCTTGGGTGTGAATGATGATTCAGGGACATCT |
| PW7 | TGAGCACAGGTTTAACAGCTAAGACCGGAGGATTACAAGAACATGACCAAC |
| PW8 | TCACGACGTTGTAAAACGACGGCCAGTGCCAAGCTTTGAACGCCAGGGATGCG TCG |
| PW9 | CATGATTACGAATTCGAGCTCGGTACCCGGGGATCCACGTTGATGCCAAACTG GGT |
| PW10 | ACTATATGAATTTTTAGCTCTTGGGTGTGATTGTCTTTGCGTCGAAGTAC |
| PW11 | TGAGCACAGGTTTAACAGCTAAGACCGGAGCACTAGTCAACCATGAGTGA |
| PW12 | TCACGACGTTGTAAAACGACGGCCAGTGCCAAGCTTGTTGCGCCGGTGCGCTC TGC |
| PW13 | CATGATTACGAATTCGAGCTCGGTACCCGGGGATCCCTTCCATCTTCTAGTGGT TC |
| PW14 | ACTATATGAATTTTTAGCTCTTGGGTGTGAATGCCTGCAATTGAACTTC |
| PW15 | TGAGCACAGGTTTAACAGCTAAGACCGGAGGAAAACACAGGCATGCTCAC |
| PW16 | TCACGACGTTGTAAAACGACGGCCAGTGCCAAGCTTACAATTTCTAGCCCAAT GAC |
| PW17 | CATGATTACGAATTCGAGCTCGGTACCCGGGGATCCTTTGCGAACTTGGAGGA GA |
| PW18 | ACTATATGAATTTTTAGCTCTTGGGTGTGAGCGTTTTCAATAGTTCGGTG |
| PW19 | TGAGCACAGGTTTAACAGCTAAGACCGGAGCCGAACAAATATGTTTGAAAG |
| PW20 | TCACGACGTTGTAAAACGACGGCCAGTGCCAAGCTTCCTCATCGAGTGGGTTC AGC |
| PW21 | TCACACCCAAGAGCTAAAAATTCATATAGT |
| PW22 | CTCCGGTCTTAGCTGTTAAACCTGTGCTCA |

### Example 2 Construction of a genome-modified strain

### 1. Construction of a strain with enhanced expression of aspartate kinase

ATCC13032 competent cells were prepared according to the classic method of Corynebacterium glutamicum (C. glutamicum Handbook, Charpter 23). The competent cells were transformed with the recombinant plasmid pK18mobsacB-Psod-lysC^{glaT3llI} by electroporation, and transformants were screened on a selection medium containing 15 mg/L kanamycin, and the gene of interest was inserted into the chromosome due to homology. The screened transformants were cultured overnight in a normal liquid brain-heart infusion medium at 30°C under shaken at 220 rpm on a rotary shaker. During this culture process, the transformants underwent a second recombination, removing the vector sequence from the genome through gene exchange. The culture was diluted in a serial gradient (10⁻² to 10⁻⁴), and the dilutions were spread on a normal solid brain-heart infusion medium containing 10% sucrose and subjected to static culture at 33°C for 48 h. Strains grown on sucrose medium did not carry the inserted vector sequences in their genome. The fragment of interest was amplified by PCR and analyzed by nucleotide sequencing to obtain the mutant strain of interest named SMCT136. In this strain, the lysC gene was mutated, the corresponding start codon was mutated from GTG to ATG, the 311th amino acid encoded by it was mutated from threonine to isoleucine, and the promoter of the lysC gene was replaced with the strong promoter Psod.

### 2. Construction of a strain with enhanced expression of homoserine dehydrogenase

The strain construction method was referred to 1. SMCT136 was used as the original strain, and pK18mobsacB-PcspB-hom^{GapG378E} was introduced into the original strain to perform modification for enhancing the expression of homoserine dehydrogenase. The obtained modified strain was named SMCT137. In this strain, the horn gene was mutated, the corresponding amino acid mutation site was G378E, and the promoter of the horn gene was replaced with the strong promoter PcspB.

### 3. Construction of a strain for 4-hydroxy-tetrahydrodipicolinate synthase promoter replacement

The strain construction method was referred to 1. SMCT137 was used as the original strain, and pK18mobsacB-ΔPdapA::mPdapA was introduced into the original strain to perform modification for 4-hydroxy-tetrahydrodipicolinate synthase promoter replacement. The obtained modified strain was named SMCT138. In this strain, the promoter of the 4-hydroxy-tetrahydrodipicolinate synthase gene was replaced with a mutated dapA gene promoter (mPdapA).

### 4. Construction of a strain for diaminopimelate dehydrogenase promoter replacement

The strain construction method was referred to 1. SMCT137 was used as the original strain, and pK18mobsacB-ΔPddh::mPdapA was introduced into the original strain to perform modification for the diaminopimelate dehydrogenase promoter replacement. The obtained modified strain was named SMCT139. In this strain, the promoter of the diaminopimelate dehydrogenase gene was replaced with a mutated dapA gene promoter (mPdapA).

At the same time, SMCT138 was used as the original strain, pK18mobsacB-ΔPddh:: mPdapA was introduced into the original strain to perform modification for diaminopimelate dehydrogenase promoter replacement. The obtained modified strain was named SMCT143.

### 5. Construction of a strain for threonine dehydratase (ilvA) promoter replacement

The strain construction method was referred to 1. SMCT137 was used as the original strain, and pK18mobsacB-ΔPilvA::mPdapA was introduced into the original strain to perform modification for the threonine dehydratase (ilvA) promoter replacement. The obtained modified strain was named SMCT140. In this strain, the promoter of the threonine dehydratase gene ilvA was replaced with a mutated dapA gene promoter (mPdapA).

At the same time, SMCT143 was used as the original strain, pK18mobsacB-ΔPilvA::mPdapA was introduced into the original strain to perform modification for threonine dehydratase (ilvA) promoter replacement. The obtained modified strain was named SMCT144.

### 6. Construction of a strain for threonine dehydratase (tdcB) promoter replacement

The strain construction method was referred to 1. SMCT137 was used as the original strain, and pK18mobsacB-ΔPtdcB::mPdapA was introduced into the original strain to perform modification for the threonine dehydratase (tdcB) promoter replacement. The obtained modified strain was named SMCT141. In this strain, the promoter of the threonine dehydratase gene tdcB was replaced with a mutated dapA gene promoter (mPdapA).

At the same time, SMCT144 was used as the original strain, pK18mobsacB-ΔPtdcB::mPdapA was introduced into the original strain to perform modification for threonine dehydratase (tdcB) promoter replacement. The obtained modified strain was named SMCT145.

### 7. Construction of a strain for citrate synthase promoter replacement

The strain construction method was referred to 1. SMCT137 was used as the original strain, and pK18mobsacB-ΔPgltA::mPdapA was introduced into the original strain to perform modification for the citrate synthase promoter replacement. The obtained modified strain was named SMCT142. In this strain, the promoter of the citrate synthase was replaced with a mutated dapA gene promoter (mPdapA).

At the same time, SMCT145 was used as the original strain, pK18mobsacB-ΔPgltA::mPdapA was introduced into the original strain to perform modification for citrate synthase promoter replacement. The obtained modified strain was named SMCT146.

The strains constructed above and their genotype information are shown in Table 2.

**Table 2 Strains and their genotypes**

| Strain Name | Genotype |
|---|---|
| SMCT136 | ATCC13032, Psod-lysC^{gla-T311I} |
| SMCT137 | ATCC13032, Psod-lysC^{gla-T311I}, PcspB-hom^{G378E} |
| SMCT138 | ATCC13032, Psod-lysC^{gla-T311I}, PcspB-hom^{G378E}, ΔPdapA::mPdapA |
| SMCT139 | ATCC13032, Psod-lysC^{gla-T311I}, PcspB-hom^{G378E}, ΔPddh::mPdapA |
| SMCT140 | ATCC13032, Psod-lysC^{gla-T311I}, PcspB-hom^{G378E}, ΔPilvA::mPdapA |
| SMCT141 | ATCC13032, Psod-lysC^{gla-T311I}, PcspB-hom^{G378E}, ΔPtdcB::mPdapA |
| SMCT142 | ATCC13032, Psod-lysC^{gla-T311I}, PcspB-hom^{G378E}, ΔPgltA::mPdapA |
| SMCT143 | ATCC13032, Psod-lysC^{gla-T311I}, PcspB-hom^{G378E}, ΔPdapA::mPdapA, ΔPddh::mPdapA |
| SMCT144 | ATCC13032, Psod-lysC^{gla-T311I}, PcspB-hom^{G378E}, ΔPdapA::mPdapA, ΔPddh::mPdapA, ΔPilvA::mPdapA |
| SMCT145 | ATCC13032, Psod-lysC^{gla-T311I}, PcspB-hom^{G378E}, ΔPdapA::mPdapA, ΔPddh::mPdapA, ΔPilvA::mPdapA, ΔPtdcB::mPdapA |
| SMCT146 | ATCC13032, Psod-lysC^{gla-T311I}, PcspB-hom^{G378E}, ΔPdapA::mPdapA, ΔPddh::mPdapA, ΔPilvA::mPdapA, ΔPtdcB::mPdapA, ΔPgltA::mPdapA |

### Example 3 Shake flask fermentation experiment of constructed strain

The engineered strain constructed in Example 2 were fermented and verified, and the specific method was as follows:
1. Medium

Seed activation medium: BHI 3.7%, agar 2%, pH 7.0.

Seed medium: Peptone 5/L, yeast extract 5 g/L, sodium chloride 10 g/L, ammonium sulfate 16 g/L, urea 8 g/L, potassium dihydrogen phosphate 10.4 g/L, dipotassium hydrogen phosphate 21.4 g/L, biotin 5 mg/L, and magnesium sulfate 3 g/L. Glucose 50 g/L, pH 7.2.

Fermentation medium: corn steep liquor 50 mL/L, glucose 30 g/L, ammonium sulfate 4 g/L, MOPS 30 g/L, potassium dihydrogen phosphate 10 g/L, urea 20 g/L, biotin 10 mg/L, magnesium sulfate 6 g/L, ferrous sulfate 1 g/L, VBl•HCl 40 mg/L, calcium pantothenate 50 mg/L, nicotinamide 40 mg/L, manganese sulfate 1 g/L, zinc sulfate 20 mg/L, and copper sulfate 20 mg/L, pH 7.2.

### 2. Fermentative production of L-threonine in a shake flask with engineered strain

(1) Seed culture: 1 loop of seed of strains SMCT136, SMCT137, SMCT138, SMCT139, SMCT140, SMCT141, SMCT142, SMCT143, SMCT144, SMCT145, and SMCT146 on the slant culture medium was picked and inoculated into a 500 mL Erlenmeyer flask containing 20 mL of seed culture medium, and cultured at 30°C and 220 r/min for 16 h.
(2) Fermentation culture: 2 mL of seed liquid was inoculated into a 500 mL Erlenmeyer flask containing 20 mL of fermentation medium and cultured at 33°C and 220 r/min under shaking for 24 h.
(3) 1 mL of fermentation broth was taken and centrifuged (12000 rpm, 2 min), and the supernatant was collected. The contents of L-threonine, lysine and isoleucine in the fermentation broth of engineered strain and control strain were detected by HPLC.

Among them, the fermentation results of the strains with preliminary threonine synthesis ability are shown in Table 3.

**Table 3 Threonine prodution in the original strain**

| Strain number | OD562 | L-threonine (g/L) | Lysine (g/L) | Isoleucine (g/L) |
|---|---|---|---|---|
| SMCT136 | 24 | 1.2 | 1.3 | 0.8 |
| SMCT137 | 23 | 2.4 | 1.1 | 1.1 |

From the results in Table 3, it can be seen that after the aspartate kinase was modified on the basis of the wild-type strain ATCC13032, the strain was able to initially accumulate threonine. With the enhancement of homoserine dehydrogenase expression, the threonine production was further improved, and 2.4 g/L of threonine could be accumulated.

The threonine production of strains in which dapA, ddh, ilvA, tdcB and gltA were individually weakened is shown in Table 4.

**Table 4 Threonine production in the individually weakened strain**

| Strain number | OD562 | L-threonine (g/L) | Lysine (g/L) | Isoleucine (g/L) |
|---|---|---|---|---|
| SMCT137 | 23 | 2.4 | 1.1 | 1.1 |
| SMCT138 | 23 | 3.0 | 0.8 | 1.2 |
| SMCT139 | 23 | 2.8 | 0.9 | 1.1 |
| SMCT140 | 22 | 3.2 | 1.1 | 0.5 |
| SMCT141 | 23 | 3.0 | 1.1 | 0.9 |
| SMCT142 | 22 | 3.2 | 1.1 | 1.1 |

The above results showed that strains SMCT138, SMCT139, SMCT140, SMCT141, and SMCT142 had a certain increase in threonine production compared to their original strain SMCT137. Using mPdapA to replace the promoters of dapA and ddh related to lysine synthesis, the lysine production was decreased by 0.3 g/L and 0.2 g/L, respectively; using mPdapA to replace the promoters of ilvA and tdcB related to isoleucine synthesis, the isoleucine production was decreased by 0.6 g/L and 0.2 g/L, respectively; and using mPdapA to replace the promoter of gltA, the threonine production was increased by 0.8 g/L.

The fermentation results of strains SMCT138, SMCT143, SMC144, SMCT145 and SMCT146 with superimposed weakening of the five genes dapA, ddh, ilvA, tdcB and gltA are shown in Table 5.

**Table 5 Threonine production of superimposed weakening strain**

| Strain number | OD562 | L-threonine (g/L) | Lysine (g/L) | Isoleucine (g/L) |
|---|---|---|---|---|
| SMCT137 | 23 | 2.4 | 1.1 | 1.1 |
| SMCT138 | 23 | 3.0 | 0.8 | 1.2 |
| SMCT143 | 22 | 3.6 | 0.4 | 1.3 |
| SMCT144 | 22 | 5.0 | 0.4 | 0.6 |
| SMCT145 | 22 | 6.4 | 0.4 | 0.3 |
| SMCT146 | 21 | 9.1 | 0.4 | 0.3 |

The above results showed that after the gradual optimization of carbon metabolic flow, the threonine production was increased from 2.4 g/L to 9.1 g/L, an increase of 279%; This indicated that after the terminal threonine synthesis pathway was unlocked, optimizing the carbon metabolic flow could significantly improve the ability of the strain to produce threonine.

Although the present invention has been described in detail above with general descriptions and specific embodiments, it is obvious to those skilled in the art that some modifications or improvements may be made based on the present invention. Therefore, these modifications or improvements made without departing from the spirit of the present invention belong to the scope of protection claimed by the present invention.

## Claims

1. A promoter having a nucleotide sequence of SEQ ID NO. 1.

2. A biological material comprising the promoter of claim 1; wherein the biological material is a recombinant DNA, a vector, or a host cell.

3. Use of the promoter of claim 1 in initiating the expression of a gene of interest.

4. Use of the promoter of claim 1 in improving the production or yield of a microbial metabolite, or in constructing a production strain for a microbial metabolite.

5. A recombinant microorganism, wherein the transcription of a gene encoding at least one enzyme selected from the following (1) to (4) is driven by the promoter of claim 1 in said recombinant microorganism:
(1) 4-hydroxy-tetrahydrodipicolinate synthase;
(2) diaminopimelate dehydrogenase;
(3) threonine dehydratase; and
(4) citrate synthase.

6. The recombinant microorganism of claim 5, wherein the enzyme activity of aspartate kinase and/or homoserine dehydrogenase in said recombinant microorganism is enhanced and/or deregulated;
preferably, the enzyme activity is enhanced by any one of or any combination of the following 1) to 6):
1) introducing a plasmid carrying a gene encoding the enzyme;
2) increasing the copy number of the gene encoding the enzyme in the chromosome;
3) altering the promoter sequence of the gene encoding the enzyme in the chromosome;
4) operably linking a strong promoter to the gene encoding the enzyme;
5) altering the amino acid sequence of the enzyme; and
6) altering the nucleotide sequence encoding the enzyme.

7. The recombinant microorganism of claim 5 or 6, wherein said recombinant microorganism is a *Corynebacterium* species; preferably *Corynebancterium glutamicum.*

8. A method for constructing the recombinant microorganism of any one of claims 5 to 7, comprising: replacing the promoter of the gene encoding at least one enzyme of 4-hydroxy-tetrahydrodipicolinate synthase, diaminopimelate dehydrogenase, threonine dehydratase, and citrate synthase with the promoter of claim 1 in a corresponding wild-type microorganism;
preferably, wherein the method further comprises: enhancing the enzyme activity of aspartate kinase and/or homoserine dehydrogenase and/or obtaining feedback-resistant aspartate kinase and/or homoserine dehydrogenase;
wherein the enzyme activity is enhanced by any one of or any combination of the following 1) to 6):
1) introducing a plasmid carrying the gene encoding the enzyme;
2) increasing the copy number of the gene encoding the enzyme in the chromosome;
3) altering the promoter sequence of the gene encoding the enzyme in the chromosome;
4) operably linking a strong promoter to the gene encoding the enzyme;
5) altering the amino acid sequence of the enzyme; and
6) altering the nucleotide sequence encoding the enzyme.

9. Use of the recombinant microorganism of any one of claims 5 to 7 in any one of:
(1) producing a microbial metabolite or a derivative thereof;
(2) selecting a strain for producing a microbial metabolite or a derivative thereof; and
(3) increasing the production and/or yield of a microbial metabolite;
preferably, wherein the microbial metabolite is threonine.

10. A method for producing threonine or a derivative thereof, comprising the step of culturing the recombinant microorganism of any one of claims 5 to 7 and isolating threonine or a derivative thereof from the culture.


### Sequence listing

<110>LANGFANG MEIHUA BIOTECHNOLOGY DEVELOPMENT CO., LTD.
<120>PROMOTER, THREONINE-PRODUCING RECOMBINANT MICROORGANISM AND USE THEREOF
<130>  KHP211124137.2
<160>  39
<170>  SIPOSequenceListing 1.0
<210>  1

<211>  222
<212>  DNA
<213>  Artificial Sequence
<400>  1
tcacacccaa gagctaaaaa ttcatatagt taagacaaca ttttggctgt aaaagacagc          60
cgtaaaaacc tcttgctcgt gtcaattgtt cttatcggaa tgtggcttgg gcgattgtta          120
tgcaaaagtt gttaggtttt ttgcggggtt gtttaacccc caaatgaggg aagaaggtag          180
aattgaactc tatgagcaca ggtttaacag ctaagaccgg ag                             222
<210>  2
<211>  46
<212>  DNA
<213>  Artificial Sequence
<400>  2
aattcgagct cggtacccgg ggatccagcg acaggacaag cactgg                         46
<210>  3
<211>  40
<212>  DNA
<213>  Artificial Sequence
<400>  3
cccggaataa ttggcagcta tgtgcacctt tcgatctacg                                40
<210>  4
<211>  40
<212>  DNA
<213>  Artificial Sequence
<400>  4
cgtagatcga aaggtgcaca tagctgccaa ttattccggg                                40
<210>  5
<211>  41
<212>  DNA
<213>  Artificial Sequence
<400>  5
tttctgtacg accagggcca tgggtaaaaa atcctttcgt a                              41
<210>  6
<211>  41
<212>  DNA
<213>  Artificial Sequence
<400>  6
tacgaaagga ttttttaccc atggccctgg tcgtacagaa a                              41
<210>  7
<211>  42
<212>  DNA
<213>  Artificial Sequence
<400>  7
tcggaacgag ggcaggtgaa ggtgatgtcg gtggtgccgt ct                             42
<210>  8

<211>  42
<212>  DNA
<213>  Artificial Sequence
<400>  8
agacggcacc accgacatca ccttcacctg ccctcgttcc ga                                42
<210>  9
<211>  46
<212>  DNA
<213>  Artificial Sequence
<400>  9
gtaaaacgac ggccagtgcc aagcttagcc tggtaagagg aaacgt                            46
<210>  10
<211>  46
<212>  DNA
<213>  Artificial Sequence
<400>  10
aattcgagct cggtacccgg ggatccctgc gggcagatcc ttttga                            46
<210>  11
<211>  40
<212>  DNA
<213>  Artificial Sequence
<400>  11
atttctttat aaacgcaggt catatctacc aaaactacgc                                  40
<210>  12
<211>  40
<212>  DNA
<213>  Artificial Sequence
<400>  12
gcgtagtttt ggtagatatg acctgcgttt ataaagaaat                                  40
<210>  13
<211>  40
<212>  DNA
<213>  Artificial Sequence
<400>  13
gtatatctcc ttctgcagga ataggtatcg aaagacgaaa                                  40
<210>  14
<211>  40
<212>  DNA
<213>  Artificial Sequence
<400>  14
tttcgtcttt cgatacctat tcctgcagaa ggagatatac                                  40
<210>  15
<211>  41
<212>  DNA
<213>  Artificial Sequence

<400>  15
tagccaattc agccaaaacc cccacgcgat cttccacatc c                41
<210>  16
<211>  41
<212>  DNA
<213>  Artificial Sequence
<400>  16
ggatgtggaa gatcgcgtgg gggttttggc tgaattggct a                41
<210>  17
<211>  46
<212>  DNA
<213>  Artificial Sequence
<400>  17
gtaaaacgac ggccagtgcc aagcttgctg gctcttgccg tcgata           46
<210>  18
<211>  56
<212>  DNA
<213>  Artificial Sequence
<400>  18
catgattacg aattcgagct cggtacccgg ggatcctccg cgacccatga attggt    56
<210>  19
<211>  50
<212>  DNA
<213>  Artificial Sequence
<400>  19
actatatgaa tttttagctc ttgggtgtga gctttgcgtt aaaagtccat       50
<210>  20
<211>  51
<212>  DNA
<213>  Artificial Sequence
<400>  20
tgagcacagg tttaacagct aagaccggag tagagcactt cggcaccgtt g     51
<210>  21
<211>  56
<212>  DNA
<213>  Artificial Sequence
<400>  21
tcacgacgtt gtaaaacgac ggccagtgcc aagcttaccc ttggcgtcct tgaccg    56
<210>  22
<211>  56
<212>  DNA
<213>  Artificial Sequence
<400>  22
catgattacg aattcgagct cggtacccgg ggatccgtat cggcagttcc agccat    56
<210>  23

<211>  50
<212>  DNA
<213>  Artificial Sequence
<400>  23
actatatgaa tttttagctc ttgggtgtga atgatgattc agggacatct                50
<210>  24
<211>  51
<212>  DNA
<213>  Artificial Sequence
<400>  24
tgagcacagg tttaacagct aagaccggag gattacaaga acatgaccaa c              51
<210>  25
<211>  56
<212>  DNA
<213>  Artificial Sequence
<400>  25
tcacgacgtt gtaaaacgac ggccagtgcc aagctttgaa cgccagggat gcgtcg         56
<210>  26
<211>  56
<212>  DNA
<213>  Artificial Sequence
<400>  26
catgattacg aattcgagct cggtacccgg ggatccacgt tgatgccaaa ctgggt         56
<210>  27
<211>  50
<212>  DNA
<213>  Artificial Sequence
<400>  27
actatatgaa tttttagctc ttgggtgtga ttgtctttgc gtcgaagtac                50
<210>  28
<211>  50
<212>  DNA
<213>  Artificial Sequence
<400>  28
tgagcacagg tttaacagct aagaccggag cactagtcaa ccatgagtga                50
<210>  29
<211>  56
<212>  DNA
<213>  Artificial Sequence
<400>  29
tcacgacgtt gtaaaacgac ggccagtgcc aagcttgttg cgccggtgcg ctctgc         56
<210>  30
<211>  56
<212>  DNA
<213>  Artificial Sequence

<400> 30
catgattacg aattcgagct cggtacccgg ggatcccttc catcttctag tggttc 56
<210> 31
<211> 49
<212> DNA
<213> Artificial Sequence
<400> 31
actatatgaa tttttagctc ttgggtgtga atgcctgcaa ttgaacttc 49
<210> 32
<211> 50
<212> DNA
<213> Artificial Sequence
<400> 32
tgagcacagg tttaacagct aagaccggag gaaaacacag gcatgctcac 50
<210> 33
<211> 56
<212> DNA
<213> Artificial Sequence
<400> 33
tcacgacgtt gtaaaacgac ggccagtgcc aagcttacaa tttctagccc aatgac 56
<210> 34
<211> 55
<212> DNA
<213> Artificial Sequence
<400> 34
catgattacg aattcgagct cggtacccgg ggatcctttg cgaacttgga ggaga 55
<210> 35
<211> 50
<212> DNA
<213> Artificial Sequence
<400> 35
actatatgaa tttttagctc ttgggtgtga gcgttttcaa tagttcggtg 50
<210> 36
<211> 51
<212> DNA
<213> Artificial Sequence
<400> 36
tgagcacagg tttaacagct aagaccggag ccgaacaaat atgtttgaaa g 51
<210> 37
<211> 56
<212> DNA
<213> Artificial Sequence
<400> 37
tcacgacgtt gtaaaacgac ggccagtgcc aagcttcctc atcgagtggg ttcagc 56
<210> 38

<211>  30
<212>  DNA
<213>  Artificial Sequence
<400>  38
tcacacccaa gagctaaaaa ttcatatagt                                        30
<210>  39
<211>  30
<212>  DNA
<213>  Artificial Sequence
<400>  39
ctccggtctt agctgttaaa cctgtgctca                                        30